# EUROPEAN PATENT SPECIFICATION

(11) **EP 3 337 897 B1**
(45) Date of publication and mention of the grant of the patent: **25.03.2020**
(21) Application number: 16770982.3
(22) Date of filing: 10.08.2016
(51) Int. Cl.: C12N 15/10

(54) **METHOD AND SYSTEM FOR ISOLATION OF NUCLEIC ACIDS**
VERFAHREN UND SYSTEM ZUR ISOLIERUNG VON NUKLEINSÄUREN
PROCÉDÉ ET SYSTÈME D'ISOLEMENT D'ACIDES NUCLÉIQUES

(30) Priority: 18.08.2015 PL 41355115
(43) Date of publication of application: 27.06.2018
(73) Proprietor: Curiosity Diagnostics sp. z o.o, 01.796 Warsaw (PL)
(72) Inventor: GEWARTOWSKI, Kamil Robert, 02-127 Warszawa (PL); MELLEM, Krzysztof, 58-560 Jelenia Góra (PL); BAJER-BORSTYN, Seweryn, 01-864 Warszawa (PL)
(74) Representative: Grzelak, Anna
(86) International application number: PCT/IB2016/054807
(87) International publication number: WO 2017/029582

(56) References cited:
- WO-A1-2008/150826
- WO-A2-2012/011091
- WO-A2-2013/111016
- US-A1- 2015 104 358
- HANYOUP KIM ET AL: "Automated Digital Microfluidic Sample Preparation for Next-Generation DNA Sequencing", JOURNAL OF LABORATORY AUTOMATION, vol. 16, no. 6, 6 July 2011 (2011-07-06), pages 405-414, XP028113037, ISSN: 2211-0682, DOI: 10.1016/J.JALA.2011.07.001 [retrieved on 2011-07-06]

## Description

### Technical field

The invention relates to a field of biochemistry, and more precisely to a method of isolation, purification, and/or concentration of nucleic acids, in particular from biological samples. It relates also to devices/systems for nucleic acid isolation according to this method.

### Background art

Isolation of nucleic acids (in particular of deoxyribonucleic - DNA, and ribonucleic - RNA, acids) from biological samples is a widely used procedure in many fields of research, diagnostics, forensics, etc. Numerous variants thereof have been developed, but what they generally have in common is a sequence of steps leading to an obtainment of a purified acid. At the starting point, nucleic acids are confined in cells of living organisms, and in eukaryotes - also in intracellular structures (including nucleus or mitochondrion). Therefore, one of the initial steps is lysis of cells with simultaneous release of their contents into solution. Certain procedures provide for subsequent removal of proteins and other substances from the solution, for instance by extraction with phenol and chloroform. In a subsequent step, released nucleic acids are separated from the solution by precipitation with a chaotropic agent and, optionally, by binding to the surface of a solid bed (e.g., filter, beads, column packing, etc.). The resulting nucleic acid precipitate is then washed to remove co-precipitated impurities. Thus purified nucleic acid is finally transferred into aqueous solution by dissolution of the precipitate, and, if the precipitate is bound to a solid bed, by elution.

Depending on the starting material, lysis may be preceded by a pre-processing step. For instance, isolation from paraffin-embedded tissue samples is preferably preceded by paraffin dissolution using mineral oil. In this case, in further processing steps a sample is used which contains, i.a., mineral oil that is removed together with aqueous solution following DNA precipitation/binding (Lin J. et al., High-quality genomic DNA extraction from formalin-fixed and paraffin-embedded samples deparaffinized using mineral oil, Anal. Biochem., vol. 395, issue 2, pp. 265-267, December 2009).

Commercially available kits for nucleic acid isolation are marketed, which contain ready-to-use lysis, precipitation, washing, and elution solutions, and columns pre-packed with nucleic acid binding bed. Bed filtration is usually supported by centrifugation of the column in a test tube.

Publication of the US patent 8816063B2 (application US13/319,855) discloses a method which comprises addition of at least one hydrocarbon to the elution solution to achieve the same elution volume in each case, especially when working with multiple batches, for example in a 48-well or 96-well plate, and with small elution volumes. It is especially advisable in vacuum-based or centrifugation-based elution, for example when using a nucleic acid binding membrane or when using nucleic acid binding particles in combination with a membrane, which serves as a barrier thereto. The applicant presumes that this goal, i.e. obtaining a more uniform eluate volumes, is achieved because the stacked layer of hydrocarbon promotes complete penetration of the eluting solution through the membrane. Preferably, the hydrocarbon is a mineral oil. However, US 8816063B2 neither mentions nor suggests the use of the at least one hydrocarbon at any of the preceding steps of the nucleic acid purification procedure, i.e. before the elution, e.g. at the step of washing. At the same time it indicates aqueous solutions with low salt concentrations, as well as water, as preferred for washing (column 8, lines 12-17 of US 8816063 B2 publication). Water is used as the washing buffer in all the examples described in US 8816063B2 (column 14, lines 46-47; column 15, line 39; column 16, line 13; column 17, line 20). A person skilled in the art will appreciate that water, as well as aqueous solutions with low salt concentrations, are used to elute nucleic acids from a nucleic acid binding phase and their use for washing poses a risk of losses of nucleic acids during washing. As is known to a person skilled in the art, the reduction of losses of purified molecules during washing is achieved through the use of washing liquids comprising organic constituents, e.g. alcohols. Such constituents must however be effectually removed (e.g. by drying the bed) before elution, if the eluate is intended for use in enzymatic reactions (see US 8816063B2, column 8, lines 17 to 24). By recommending the use of water and aqueous solutions with low salt concentrations for washing, as well as by using water as the washing buffer in the examples described in US 8816063B2, the applicant gives evidence of lacking an effectual method of purifying the bed from the washing liquid.

Publication US2013/0274454A1 (application US 13/834,901) concerns retrieval of a liquid from a porous solid phase, comprising a step of forming a complete layer of another liquid, immiscible with the liquid bound to the solid phase, across the surface of the liquid bound to the solid phase at the first end of the said solid phase, and a step of applying a pressure differential across the porous solid phase to cause the immiscible liquid to move therethrough towards a second end of the phase. The liquid initially bound to the porous solid phase is thereby displaced towards and released from the second end thereof. The solution described in US2013/0274454A1 applies to isolation of nucleic acids, during which it is used to retrieve an eluate from a bed. The immiscible liquid described in US2013/0274454A1 (which may be a mineral oil) is not used at any of the steps of the procedure prior to elution. Moreover, US2013/0274454A1 also describes problems arising from incomplete removal of chaotropic agents and organic solvents - constituents of effective washing liquids - from bed prior to elution (see US2013/0274454A1 par. [0035]). As a solution to these problems only the use of an alternative method, described by Hourfar et al., avoiding the use of organic constituents in washing liquids, is indicated. A person skilled in the art will appreciate that this will cause losses of nucleic acids during washing. To still retrieve an amount of nucleic acids from bed during elution, it is carried out at a temperature as high as 80°C (US2013/0274454A1 par. [0035]). Hence the disadvantage of this method is not only the substantial loss of nucleic acids during washing, but also the necessity of energy-consuming heating to retrieve their remains, as is described in the examples (see Example 5 of US2013/0274454A1).

Recent decades have witnessed significant efforts aiming at simplification and reduction of time consumption of the procedure for nucleic acid isolation (Boom R., Rapid and simple method for purification of nucleic acids, J. Clin. Microbiol., vol. 28, issue 3, pp. 495-503, March 1990), as well as its miniaturisation and automation. When the process is carried out in microfluidic systems (instead of traditional Eppendorf test tubes), nucleic acids from samples of very small volume can be purified, simultaneously minimising the reagent consumption. The automation allows for simultaneous isolation of nucleic acids from multiple samples, while reducing human labour and assuring high reproducibility.

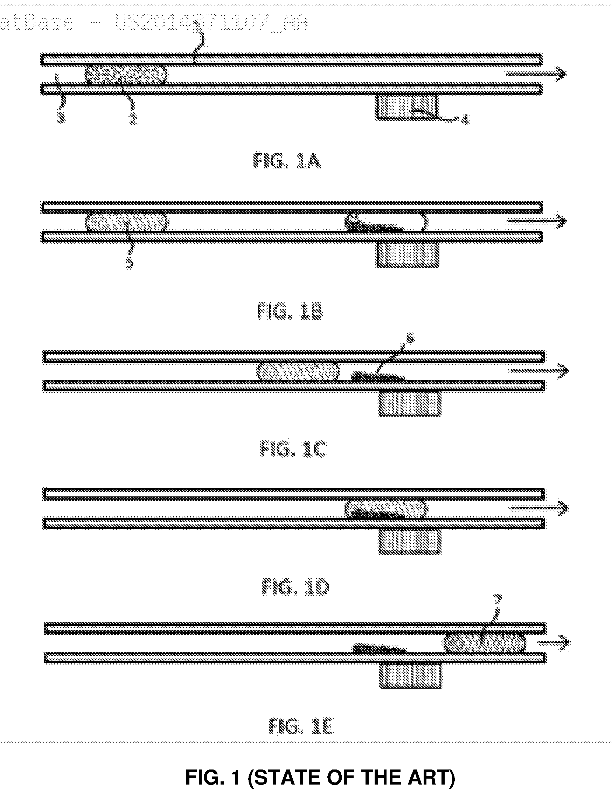

The US 14/374,727 patent application discloses systems and methods for isolating biomolecules within conduits. In one embodiment (par. [0034]), the method comprises the use of a capillary tube, a pump and a localised magnet field at a location along the length of the capillary tube. First a slug of a bead-mixture, which includes a buffer and beads with a biochemistry coating, and the target biomolecule, is drawn into the capillary tube. The beads may be magnetic beads with a biochemistry coating or non-magnetic beads (silica, ceramic, a polymer, etc.) with a paramagnetic coating. This is followed by a slug of immiscible fluid, for example air or oil, and then followed by discrete slugs of ethanol, air, oil and elution buffer. The slugs flow within the tube passing the localised magnetic field, where upon the paramagnetic beads are trapped within the magnetic field, while the other components of the bead-mixture slug continue to flow along the tube, removing all the unbound molecules from the paramagnetic beads. The continuous flow of slugs next brings an oil or air slug, which is used as a buffer to prevent mixing of the bead-mixture slug with the ethanol slug. The ethanol slug cleans any remaining contaminants from the paramagnetic beads. The cleaning step may be repeated. After the ethanol slug has passed, an oil buffer passes prior to the slug of elution buffer to prevent any trace elements of ethanol along the tube from mixing with the elution buffer slug. The elution buffer then flows over the paramagnetic beads, releasing the biomolecule targets from the paramagnetic beads into the elution buffer slug. Selected steps of the process are illustrated in Fig. 1 (A to E). The oils used for generating immiscible phases can include and are not limited to silicone oil, perfluorocarbon oil, and perfluoropolyether oil (par. [0046]). In a purification example, DNA was mixed by pipetting with magnetic beads and aspirated into a PTFE capillary tube. The beads and bound target DNA were removed from the bead-DNA suspension to the wall of the capillary as the suspension passed a magnet. The ethanol slugs passed over the so fixed DNA-bead pellet, washing the pellet and removing remaining contaminants. The air and oil slugs were then delivered past the pellet to remove residual ethanol. In the final step of the purification process, the elution buffer slug eluted the target DNA from the beads into solution as it passed the bead pellet. The fluids were pumped at a constant volume flow rate of 10 µl/min (par. [0152]). A person skilled in the art will easily calculate that the entire procedure lasted for at least over 11 minutes, and only the drying of the bed to remove ethanol as a washing fluid took one minute.

Though automated in part, the above procedure still requires that the sample is manually lysed biologically and mixed with beads in a manual process prior to aspiration into a capillary tube. It does not simplify the method of delivery of working fluids onto the bed either. The use of a paramagnetic bed for nucleic acid binding requires that a magnet is used as a component of the equipment setup.

In addition, in the procedure described above, the bed beads immobilised within the conduit form a pellet on the conduit wall (Fig. 1). The fluids passing the conduit flow mostly over the pellet washing it's surface only. This is related to a reduced exposure of the particles immobilised on the beads to the fluids used in the procedure, and thus to a less thorough washing, because the washing buffer weakly penetrates the bead pellet. All the more a hydrophobic fluid, such as oil, because of a difference in polarity, will not penetrate the inside of the pellet filled with a hydrophilic alcohol solution, which implies that the pellet must be dried with air. Omission of the bed exposure to air would likely result in residual ethanol remaining in the pellet. For the same reasons the elution efficiency is reduced as well. This problem is known in the state of the art, for instance in many commercial nucleic acid isolation kits making use of beads in the form of magnetic balls, where it is advised to break magnet operation during washing and elution, to suspend the balls in solution and collect them again using a magnet (for example MagJET kits from Life Technologies), in order to enhance the penetration of washing and elution fluids between the beads.

In addition, a solution like the one reported in the above discussed US 14/374,727 patent application requires a relatively slow liquid flow, which does not result in the deposited bed beads being washed away by the fluid flowing over them. Therefore, the solution is not suitable for use in fast nucleic acid isolation/purification procedures.

Another example of nucleic acid purification in conduits is found in publication US2015/0104358A (patent application US 14/510,359). Disclosed is a nucleic acid extraction device having a tube subjected to an antistatic treatment, in which a first plug formed of an oil, a second plug formed of a washing liquid which does not mix with an oil, a third plug formed of an oil, a fourth plug formed of an eluate which does not mix with an oil, and a fifth plug formed of an oil are arranged in this order. The nucleic acid binding bed comprises magnetic beads and a magnet is used to move it through the plugs. Also here, similarly to US 8816063B2 and US US2013/0274454A1 discussed above, the use of water or low concentration (most preferably below 5mM) Tris-HCI solution, optionally containing EDTA, as a washing buffer is recommended (see US2015/0104358A1 par. [0052]). To avoid losses of target molecules, which are foreseeable with such washing buffers, the applicant avoids stirring the beads with the washing buffer (see par. [0151]), which decreases the efficiency of washing. The applicant does not disclose the yield of such procedure, but assays the potential for increasing the amount of DNA retrieved from the bed by increasing the elution temperature (see par. [0169] to [0176]).

The above examples show that in the methods hitherto used for the isolation and/or purification of nucleic acids, either the use of effective washing solutions was avoided, for the lack of an effective method for their removal from bed, which led to losses of nucleic acids during washing and the necessity of energy-consuming heating during elution, or washing liquids were removed from bed by means of time-consuming drying.

The goal of the inventors of the present solution is to overcome the shortcomings of the state of the art, and further to simplify and automate the nucleic acid isolation procedure within conduits, and also to significantly reduce time and costs required to carry it out. The purpose is to obtain, in the nucleic acid isolation procedure, a material of a purity that allows for the carrying out of enzymatic reactions, with high yield, in the shortest possible time, with the lowest labour input and at minimum cost.

The above goals are achieved by developing a new flow method and system for isolating, purifying, and concentrating nucleic acids, based on the use of a hydrophobic fluid, which passes through a bed placed in the lumen of a conduit, stationary relative to the conduit and occupying the entire cross section of the conduit. Unexpectedly, the inventors have observed that in the method and system according to the invention, the hydrophobic fluid effectively washes out from the bed residual fluids used earlier, in particular the washing fluids, thus eliminating the time-consuming drying of the bed, while not washing out (not eluting) the nucleic acids from the bed, so that the efficiency of the nucleic acid isolation and/or purification and/or concentration is not reduced (as compared with the standard methods, where - to remove the washing fluids - the bed is brought into contact with air only), even when a very low number of isolated and/or purified and/or concentrated molecules is bound to the bed.

### Disclosure of the invention

In the first aspect, the invention relates to a method of isolation and/or purification and/or concentration of nucleic acids, in particular from biological samples, performed in flow within a conduit, comprising the steps of nucleic acid binding on a bed, bed washing and elution of the bound nucleic acids, wherein the nucleic acids are being bound to a bed placed in the lumen of the conduit, stationary relative to the conduit and occupying the entire cross section of the conduit, and the working fluids, including at least a washing fluid and an elution fluid, are passed through the bed in portions separated from each other by a hydrophobic fluid immiscible with them, and wherein hydrophobic fluid is selected from mineral oil, silicone oil, hexadecane, paraffin oil, fluorinert and mixtures thereof, and wherein the hydrophobic fluid, while passing through the entire bed, washes out the residual preceding fluid, in particular the washing fluid, wherein the flow in the conduit is provided by conveying means.

A sample comprising nucleic acids is delivered into a conduit, preferably a capillary conduit or a microfluidic channel, in particular on a chip or microchip, for example in front of the bed.

If the target nucleic acids to be isolated according to the method are contained in structures surrounded by biological membranes, for example in cells or organelles, the sample is preferably lysed. It should be noted that the sample can be, for example, mixed with a lysis fluid prior to being delivered to the conduit, or it can be, for example, mixed with a lysis fluid in the conduit. The sample can be also lysed with another method, and a person skilled in the art will be able to select a method for lysis suitable for a given case. The conduit may also be supplied with a previously lysed sample or with such one, wherein the nucleic acids are in solution, and are not surrounded by biological membranes.

After the sample delivery, the following is passed through the bed in the order as follows:
- the sample comprising released nucleic acids in the form able to bind to the bed,
- then, optionally, a portion of a hydrophobic fluid,
- then other working fluids, including at least:
   - washing fluid or fluids,
   - a portion of hydrophobic fluid, as needed to remove residual washing fluids from the bed,
   - elution fluid,
- optionally the last portion of hydrophobic fluid.

It is to be noted that the flow of the portion of the hydrophobic fluid following the washing fluids, which flows through the entire lumen of the bed, entirely removes residual washing fluids from the bed, including residual contaminants remaining in the bed, so that the drying of the bed is not necessary and it is possible to elute nucleic acids directly after this step.

The product of the isolation/purification/concentration is collected at one end of the conduit.

It is to be noted that the present method does not comprise the bed drying step following the passing of the washing fluid. This step, which is present in the methods known from the state of the art, being time consuming and often troublesome because of the necessity of, for example, additional centrifugation, is not required in the method according to the present invention, because the portion of hydrophobic fluid, passing through the bed and not only washing its surface, effectively washes out the residual washing fluid or fluids. The method according to the present invention overcomes the shortcomings from the state of the art also in this respect that at any stage it does not require the use of a magnet nor any other manipulations with the bed. The solution with nucleic acids for purification, isolation and/or concentration can be delivered to the conduit without any additional mixing, for example with magnetic beads. In addition, in the method according to the present invention all the working fluids, flowing through the entire cross section of the conduit, are passed through the bed that also occupies the entire cross section of the conduit, thus providing a very effective washing and elution. The method according to the present invention allows also for significantly (e.g., twenty times) larger flow rates of the working fluids and significant acceleration of the procedure.

The present application provides thus a method of isolation and/or purification and/or concentration of nucleic acids, which is characterised by a maximum simplicity of operations, and minimum time and labour input.

In a preferred embodiment, the hydrophobic fluid is a mineral oil and/or silicone oil.

A person skilled in the art will appreciate that other hydrophobic fluids can also be used in the method according to the present invention, provided that they are immiscible with the other working fluids used in the procedure. Examples of other hydrophobic fluids for use in the invention are, for instance, hexadecane, paraffin oil and/or fluorinert.

In an embodiment, the conveying means are provided by overpressure provided from the first end of the conduit, especially in the form of a pump, preferably a syringe pump, and/or by underpressure provided from the second end of the conduit.

In an alternative embodiment, the conveying means are provided by centrifugation, wherein the first part of the conduit is located the closest to the axis of rotation.

In embodiments, silica membranes from commercial minicolumn kits for purification of nucleic acids (Genomic Mini from A&A Biotechnology, Viral RNA Mini Kit from Syngen, and QIAamp DNA Mini Kit from Qiagen) are used as the bed. Other embodiments use other nucleic acid binding materials as the bed, preferably glass, borosilicate and/or silica materials, in amorphous form, as fibres, particles, beads, granules, or any other form, advantageous for binding of nucleic acids.

The invention relates also to a system for isolation and/or purification and/or concentration of nucleic acids with the method according to the present invention, in particular from biological samples, said system comprising:
- a conduit or conduits, preferably capillary or microfluidic conduit or conduits, wherein the conduit comprises a first and a second part of the conduit,
- conveying means, and
- a nucleic acid binding bed placed in the lumen of the conduit between the first part of the conduit and the second part of the conduit, stationary relative to the conduit and occupying its entire cross section.

The system uses working fluids for purification, isolation and/or concentration of nucleic acids, the working fluids including at least a washing fluid or fluids and an elution fluid, wherein said fluids are used in portions separated from each other by a hydrophobic fluid immiscible with them, and said hydrophobic fluid, wherein the hydrophobic fluid is selected from mineral oil, silicone oil, hexadecane, paraffin oil, fluorinert and mixtures thereof.

In an embodiment, the first part of the conduit represents a reservoir for working fluids, and said working fluids, including at least a washing fluid, a hydrophobic fluid immiscible with the washing fluid, and an elution fluid immiscible with the hydrophobic fluid, can be placed together in a working reservoir, preferably located in the first part of the conduit, more preferably at the first end of the conduit, in the form of portions, separated from each other by the hydrophobic fluid immiscible with them. In a particular case, the working reservoir is the first part of the conduit, which allows for the manufacture of a system in the form of conduits ready for use in the method according to the present invention after application of a sample.

In addition, the system comprises means for delivering a sample, comprising nucleic acids, to a location inside the conduit in front of the bed and behind or within the working reservoir. In one of the embodiments, allowing for a two-way flow through the bed, the means for delivering the sample may allow for delivering the sample behind the bed. The means for sample delivery may be, for example, a suitably located opening in the conduit and/or in the working reservoir.

The system according to the present invention comprises conveying means that can be used to pass through the bed a sample comprising nucleic acids, preferably released, i.e. not surrounded by biological membranes - in the form able to bind to the bed, then optionally a portion of a hydrophobic fluid, then subsequent working fluids, including at least a washing fluid and an elution fluid, in the form of portions, separated from each other by a hydrophobic fluid immiscible with them, and said hydrophobic fluid, wherein the hydrophobic fluid is selected from mineral oil, silicone oil, hexadecane, paraffin oil, fluorinert and mixtures thereof, and subsequently optionally the last portion of a hydrophobic fluid.

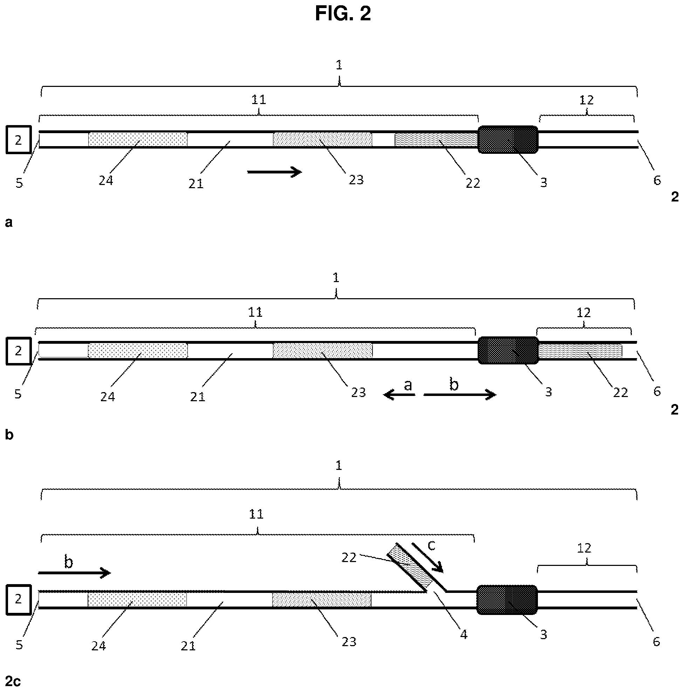

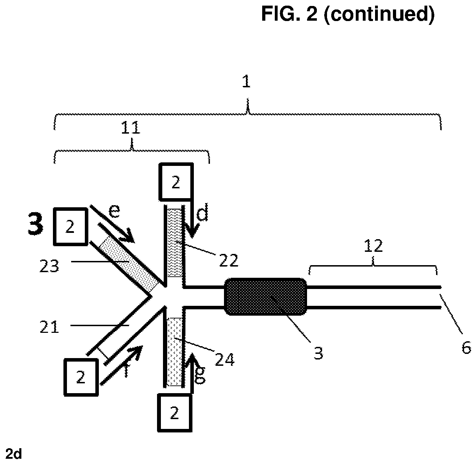

A scheme of a few variants of the system according to the present invention is shown in Fig. 2 a-d. It is evident for a person skilled in the art that the features of the system indicated in each variant may be combined with each other. In an embodiment, the system according to the present invention may enable one-way flow through the bed, through which working fluids are passed in sequence, in portions separated by a hydrophobic fluid immiscible with them, including in particular a washing fluid or fluids and an elution fluid separated from each other by a hydrophobic fluid, immiscible with them, which washes out the residual washing fluid or fluids from the bed (Fig. 2a).

In another embodiment, the system according to the present invention may enable two-way flow through the bed, for example so that a sample comprising nucleic acids, lysed if required, is delivered behind the bed, passed through the bed in one direction, and then again in the opposite direction, and subsequently other working fluids are passed through the bed in portions separated from each other by a hydrophobic fluid immiscible with them, including in particular a washing fluid or fluids and an elution fluid separated from each other by a hydrophobic fluid immiscible with them, which washes out residual washing fluid or fluids from the bed (Fig. 2b). This embodiment has an additional advantage as it allows for more than one passing of the sample through the bed, thus allowing for more efficient binding of nucleic acids to the bed.

In another embodiment, the system according to the present invention allows for delivering a sample comprising nucleic acids in front of the bed, through an opening, preferably through a side conduit. Such a side conduit may represent means for sample delivery (Fig. 2c).

In yet another embodiment, the system according to the present invention allows for delivering a sample and individual working fluids through separate conduits. A person skilled in the art will notice that, with such solution, one can account for valves controlling flows of the fluids, so as to prevent their undesired mixing. The conduits supplying working fluids may deliver the working fluids at different locations relative to the bed, preferably deliver the working fluids to a single point in front of the bed (Fig. 2d).

It is to be noted that in embodiments of the system according to the present invention, the conduit walls will be wetted by a thin film of hydrophobic fluid, preferably oil, on which subsequent portions of solutions will be moving. The research carried out by the inventors of the present invention reveals that in spite of the fact that at any time, the solution and a small quantity of oil flow simultaneously through the bed, it does not affect negatively the operation of the system and no effect of the phenomenon on the isolation efficiency has been observed.

In addition, the inventors noticed that the quantity of a hydrophobic fluid, for example a mineral oil, in the eluate can be significantly reduced by passing the eluate through a clean conduit, non-coated with oil. It is so because the undesired hydrophobic fluid, for example mineral oil, unlike water adheres to the walls of the conduit, for example a conduit made of PTFE (polytetrafluoroethylene, Teflon).

It is to be noted that the eluate may consequently contain a small quantity of oil. The oil can be centrifuged or the resulting eluate can be mixed, for example using ultrasound, to obtain emulsion for further processing. The experience of the inventors indicates, however, that it has no effect on enzymatic reactions with nucleic acids so isolated, in particular on the course of a PCR.

In a preferred embodiment, the hydrophobic fluid is a mineral oil and/or silicone oil.

A person skilled in the art will notice that other hydrophobic fluids can also be used in the system according to the present invention, provided that they are immiscible with the other working fluids, wherein the hydrophobic fluid is selected from mineral oil, silicone oil, hexadecane, paraffin oil, fluorinert and mixtures thereof.

In an embodiment, the conveying means are provided by overpressure provided from the first end of the conduit, especially in the form of a pump, preferably a syringe pump, and/or underpressure provided from the second end of the conduit.

In an alternative embodiment, the conveying means are provided by centrifugation, wherein the first part of the conduit is located the closest to the axis of rotation.

The method and system according to the present invention allow for storage of all the fluids (reagents, washing fluids, etc.) needed in the course of procedure, except for the sample, in a single working reservoir, and for using them by performing a single, continuous or interrupted, conveying movement, at constant or variable speed, provided by conveying means (preferably by application of a pressure difference, but application of centrifugation is also possible), pushing these fluids from the said reservoir, with no additional fluid transport operations (for example pipetting). Therefore, the required operations are confined to (1) delivery of the sample comprising nucleic acid for isolation, and (2) conveying the working fluids - as opposed to the method known in the prior art, disclosed for example in the US 8,816,063 patent, comprising much more operations, requiring a larger number of devices. The use of a stationary (i.e. fixed within the conduit, through which the fluids flow, for the entire duration of the procedure) bed to bind nucleic acids eliminates the necessity to perform any operations on the bed (such as for example the use of a magnet, as necessary in the case of a bed of paramagnetic beads, as disclosed in the US 14/347,727 patent application). In addition, in the method according to the present invention, individual working fluids (portions, for example of volumes corresponding to a single isolation) are separated from each other by a hydrophobic fluid immiscible with them, preferably a mineral oil and/or silicone oil. Following the passing of a washing fluid through the bed, where the nucleic acids from a sample have previously been bound, a hydrophobic fluid is also being passed, preferably a mineral oil or silicone oil, which separates said fluid from the eluent following the fluid, and prevents the washing fluid and the eluent from mixing, and also effectively washes out the residual washing fluid from the bed, making the drying step unnecessary. The present inventors have observed unexpectedly that the passing of a mineral oil through the bed with nucleic acids bound to it does not simultaneously result in reduced efficiency of the nucleic acid elution following directly this step, i.e., the quantity of nucleic acids eluted after such an operation is not significantly smaller than in the standard procedure, where no liquid passes through the bed after last washing, and before elution. Moreover, passing a hydrophobic fluid through the bed, comprising bound nucleic acids, after the washing step and before the elution step brought an unexpected advantage of eliminating the necessity of drying the bed from residual washing fluid, as necessary in the methods known in the state of the art. The residual washing fluid, as is known to those skilled in the art, interferes with enzymatic reactions (especially PCR), that are carried out on isolated nucleic acids, and therefore must be removed by drying the bed. Elimination of the necessity of drying the bed in the method according to the present invention brought an unexpected advantage of significantly shortening the duration of the procedure and simplifying it.

The advantage of the method and the system according to the invention is also the speed of procedure. The final quantity of eluted biomolecules, in particular of nucleic acid, is the same, irrespective of the method used and the concentration of target molecules, however, the time needed to carry out purification with the method according to the present invention and the methods known from the prior art is radically different. For example, purification of DNA with a standard minicolumn method takes approximately 10 minutes, with the flow method disclosed in the US 14/347,727 patent application - more than 11 minutes, whereas the method according to the invention takes approximately one and a half minute only. This is related to the possibility of using, in the method according to the invention, the volume flow rate of the order of one millilitre per minute, whereas in the flow method from the state of the art, disclosed in the US 14/347,727 patent application, it is 10 microliter per minute only. The flow rate of fluids in the method according to the invention is about twenty times higher than in the said flow method known from the state of the art.

The system according to the invention comprises a conduit, preferably a capillary or a microfluidic one. The first part of the conduit represents preferably a reservoir for working fluids, and in an embodiment of the invention may be connected with conveying means (for example, a syringe pump). Another embodiment provides a connection of underpressure to the second end of the conduit, behind the bed. In yet another embodiment of the invention, centrifugation of the entire system is possible, wherein the first part of the conduit is located the closest to the axis of rotation.

In an embodiment, the first or the second part of the conduit of the system according to the invention comprises an opening in the wall of the conduit used to deliver the sample into the conduit. Between the first and the second part of the conduit there is a nucleic acid binding bed (for example, a membrane, a filter, beads immobilised between partitions, etc.), stationary relative to the conduit and occupying its entire cross section, so that all fluids flowing between the first and the second part of the conduit flow through the bed, and not e.g. over or next to it, as in solutions known from the prior art, for example those disclosed in the US 14/347,727 patent application.

In an embodiment, the first part of the capillary conduit is filled with a hydrophobic fluid, preferably mineral oil and/or silicone oil, wherein portions of working fluids immiscible with the hydrophobic fluid are suspended: optionally a lysis fluid, and a washing fluid and an eluent - in the given order, counting from the side of the nucleic acid binding bed and the sample supply point. Following the sample delivery, for example through an opening in the wall of the conduit, the sample can mix with the lysis fluid. As a result, the cells in the sample are lysed (broken up) and their contents, including nucleic acids, released, , into solution. Subsequently, the fluid resulting from the mixing of the lysis fluid with the sample is pushed, as first one, through the nucleic acid binding bed due to operation of the conveying means (for example syringe pump, underpressure, centrifugation, etc.). As a result, the nucleic acids from said fluid are bound on the bed. Then, a portion of a hydrophobic fluid, preferably a mineral oil and/or silicone oil, separating the lysis fluid from the washing fluid and preventing these fluids from mixing with each other, is passed through said bed. Following the portion of the hydrophobic fluid, the washing fluid flows through the bed removing contaminants from the bed. Then, the hydrophobic fluid, preferably mineral oil and/or silicone oil, flows again through the bed, removing the residual washing fluid from the entire bed volume. The penultimate step is the passing of the eluent through the bed, whereby the said eluent elutes the nucleic acids from the bed, yielding the product of the isolation process. Finally, the hydrophobic fluid, preferably a mineral oil and/or silicone oil, optionally passes through the bed again, thus supporting the retrieval of the entire eluate volume from the bed.

As used here, the terms "nucleic acids" or "nucleic acid" relate essentially to a molecule or molecules comprising one or more subunits of nucleic acids. A nucleic acid may comprise one or more subunits selected from adenosine (A), cytosine (C), guanine (G), thymine (T) or uracil (U). In particular, a nucleic acid is DNA and/or RNA.

As used here, the term "purification" relates to any process comprising the release of a given molecule from foreign, external or undesired elements or to reduction of the quantities of such elements.

A "conduit" in the meaning of the present invention is a channel or another route that can be used for transporting molecules, in particular fluids, wherein the term "fluids" comprises both liquids (including solutions, suspensions, etc.) and gases. A "conduit" may be preferably a capillary conduit, a channel or conduit on a chip or a microchip, and/or another means forming a route for transporting fluids and/or solid particles in processes employing microfluidics. Preferably, a "conduit" should be manufactured from a hydrophobic material, which prevents hydrophilic solutions from mixing by sticking to the conduit walls when the fluids are moving.

A "hydrophobic fluid" in the meaning of the invention is any fluid immiscible with the washing fluid and the elution fluid. It may be a liquid or a gas. In a preferred embodiment, the hydrophobic fluid is a mineral oil, silicone oil, hexadecane, paraffin oil, fluorinert or their mixtures.

"Conveying means" in the meaning of the invention are any means allowing for maintaining a controlled flow of fluids through the conduit, so that the conveying means may be overpressure and/or underpressure provided from the proper end of the conduit, a suction and force pump, a syringe pump, application of the action of centrifugal force, for example by centrifugation, etc. For a person skilled in the art it is evident that the conveying means can be applied to one (the first or the second) end of the conduit or to both ends of the conduit, and for a system with supply of individual fluids by separate conduits, also to multiple ends of supply conduits.

A person skilled in the art will notice that the system and method according to the invention can find application in various types of processing of biomolecules, not only nucleic acids, including purification, isolation or concentration of such molecules.

Below, the invention will be explained through detailed embodiments thereof, which are however non-limiting to its scope, with reference to the accompanying figures, wherein:

### Brief description of Figures

**Fig. 1** shows the scheme of a system for isolation of biomolecules, in particular nucleic acids, in conduits, as known from the state of the art.
**Fig. 2** shows the scheme of a system for isolation of nucleic acids according to the invention in various embodiments; Fig. 2a shows the scheme of a system with one-way flow through the bed, Fig. 2b shows the scheme of a system with two-way flow through the bed, Fig. 2c shows the scheme of a system with sample supply through a side conduit, Fig. 2d shows the scheme of a system with supply of individual fluids through separate conduits.

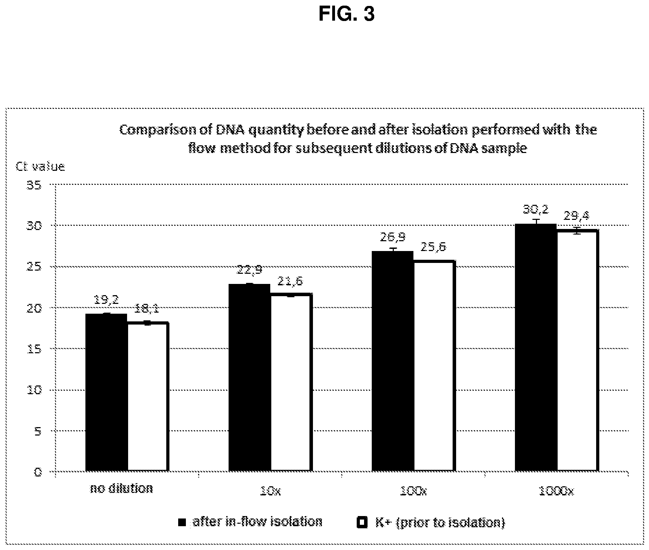

**Fig. 3** shows the results of quantitative analysis of DNA isolated with the method according to the invention, before isolation according to the invention (empty bars) and after the isolation (filled bars). The amount of DNA is expressed by the threshold cycle (Ct) value (called also quantification cycle) of quantitative PCR, which decreases with increasing quantity of DNA in the sample.

### Examples

### Example 1

### System for DNA isolation

In an embodiment of the system according to the invention, as shown schematically in Fig. 2a, the system for isolation/purification/concentration of nucleic acids comprises a conduit 1, preferably a capillary conduit. The first part 11 of the conduit 1 is a reservoir for working fluids, and in an embodiment of the invention may be connected with conveying means 2 (for example a syringe pump, not shown in Fig. 2a, only its location is indicated).

Another embodiment (not shown in Fig. 2) provides connection of the conveying means 2 in the form of underpressure applied to the second end 6 of the conduit 1, behind the bed 3. Yet another embodiment (not shown in Fig. 2) allows for centrifugation of the entire system, wherein the first part 11 of the conduit 1 is located the closest to the axis of rotation.

A sample 22, if necessary lysed, may be supplied to the first part 11 of the conduit 1 in front of the bed 3. Between the first part 11 and the second part 12 of the conduit 1 there is a nucleic acid binding bed 3 (for example, a membrane, a filter, beads immobilised between partitions, etc.), stationary relative to the conduit 1 and occupying its entire cross section, so that all fluids flowing between the parts 11, 12 of the conduit 1 flow through the bed, and not, for instance, over or next to it.

In an embodiment of the invention, the first part 11 of the capillary conduit is filled with a hydrophobic fluid 21, preferably a mineral oil, with portions of working fluids immiscible with the hydrophobic fluid suspended therein: a washing fluid 23 and an elution fluid 24 - in the given order, counting from the side of the nucleic acid binding bed 3 and the sample 22 supply point. The sample 22 may be delivered, for example, through an opening, wherein said opening may be located in the wall of the conduit 1. In another embodiment of the system, the sample supply opening is the first end 5 of the conduit. If required, before delivering or after delivering, the sample is lysed using any method (for example with ultrasound or a suitable lysis fluid). As a result, cells/organelles in the sample are lysed (broken up) and their contents, including nucleic acids, are released into solution. After delivering the sample 22, the fluid comprising the sample 22 is passed, as first one, through the nucleic acid binding bed 3 due to operation of the conveying means 2 (for example, a syringe pump placed as shown in Fig. 2a, or underpressure, etc.). As a result, the nucleic acids from said fluid are bound to the bed 3. Then, preferably, the hydrophobic fluid 21 flows through the bed. Subsequently, the washing fluid 23 flows through the bed 3, removing contaminants from the bed. The washing step may be repeated, for example using several portions of the washing fluid 23. Then, the hydrophobic fluid 21, preferably a mineral oil, flows through the bed 3, removing the residual washing fluid 23 from the entire volume of the bed 3. Due to this solution the bed 3 does not need to be dried before the elution of the nucleic acids. Then, the elution fluid 24 passes through the bed 3, and elutes the nucleic acids from the bed 3, yielding the isolation product that can be received at the second end 6 of the conduit 1. In addition, finally the hydrophobic fluid 21, preferably a mineral oil, optionally passes again through the bed 3, thus supporting the retrieval of the entire eluate volume from the bed 3.

In another variant of the system, shown in Fig. 2b, the system according to the present invention allows for two-way flow through the bed 3. The sample 22 comprising nucleic acids, lysed if required, may be delivered behind the bed 3. In the course of isolation/purification/concentration, the sample 22 is passed through the bed 3 in one direction a, and then again in the opposite direction b, and subsequently other working fluids are passed through the bed in portions separated from each other by a hydrophobic fluid 21 immiscible with them, which washes the residual washing fluid or fluids 23 out from the bed. This embodiment has an additional advantage of allowing for more than one passing of the sample 22 through the bed 3, thus allowing for more efficient binding of nucleic acids to the bed. The sample 22 is delivered to the conduit 1 through an opening located in front of the bed 3 from the side of the second end 6 of the conduit 1 (not shown in Fig. 2b), or directly through an opening being the second end 6 of the conduit.

In another embodiment, shown in Fig. 2c, the system according to the present invention allows for delivering the sample 22 comprising nucleic acids in front of the bed 3 through an opening 4 in the wall of the conduit 1, for example through a side conduit in direction c. In this solution, the side conduit delivering the sample 22 in direction c is the means of delivering the sample 22. Subsequently, the working fluids 23, 24, in portions separated from each other by the hydrophobic fluid 21 immiscible with them, which washes the residual washing fluid or fluids 23 out from the bed, are passed in turn through the bed 3 in direction b, preferably preceded by the hydrophobic fluid 21.

In yet another embodiment, shown in Fig. 2d, the system according to the present invention allows for delivering a sample 22, as well as individual working fluids 23, 24, and the hydrophobic fluid 21 immiscible with them, through separate conduits. In such solution, the system preferably comprises valves controlling flows of the fluids (not shown in the figure), which prevent the fluids from undesired mixing. In the presented variant, the conduits supply the sample 22, the washing fluid 23, the hydrophobic fluid 21, and the elution fluid 24, in appropriate order, in directions d, e, f, and g to a single point in front of the bed 3 (Fig. 2d). These fluids are passed in appropriate order through the bed 3 towards the second end 6 of the conduit.

### Example 2

### Analysis of efficacy of DNA isolation with the method according to the invention

### 2.1. Materials and methods

The isolation of DNA for the purposes of this analysis was carried out with the following minicolumn kits: Genomic Mini from A&A Biotechnology, Viral RNA Mini Kit from Syngen, and QIAamp DNA Mini Kit from Qiagen. RNA was isolated with Syngen Viral RNA Mini Kit. All control isolations were carried out in line with recommendations of the manufacturers. The isolations testing if the hydrophobic fluid can be used to improve the isolation were carried out either with minicolumns using a centrifuge or in the system according to the present invention (i.e., with the flow method, according to Fig. 2a), where the nucleic acid binding bed (from commercial kits), immobilised in a Teflon minicolumn, was serially connected with the first part 11 of the conduit , so that the walls of the minicolumn were the walls of the conduit within this section, the minicolumn bed was the bed 3 immobilised in the lumen of the conduit, and the minicolumn inlet was located at the side of the first part 11 of the conduit. The sample and the working fluids, i.e., the solutions from the kit, were delivered in appropriate order, in portions separated from each other by portions of a mineral oil, to the first part 11 of the conduit 1. Pure plasmid DNA, genomic DNA from *E. coli*, *M. smegmatis*, or *B*. *subtilis* cells, and lentiviral DNA were used in tests. Pure *in vitro* transcribed RNA was used in tests with ribonucleic acid. The quantitative DNA assays were carried out with real-time PCR method using a LightCycler 480 II (Roche) and SensiFAST SYBR No-Rox (Bioline) reagents. A three-stage PCR program had the following course: initial denaturation: 95°C for 5 min, amplification: 45 cycles: 15 s 95°C, 25 s 55°C, 15 s at 72°C. Quantitative RNA assays were carried out with reverse transcription and real-time PCR method using a LightCycler 480 II (Roche) and OneStep SensiFAST SYBR No-Rox (Bioline) reagents. The PCR program had the following course: reverse transcription at 40°C for 2 min; initial denaturation: 95°C for 5 min, amplification: 45 cycles: 15 s 95°C, 25 s 55°C, 15 s at 72°C.

### 2.2. DNA isolation

### Mineral oil passed through a minicolumn for DNA isolation has no effect on the purification efficiency

The quantity of plasmid DNA purified with a QIAamp DNA mini kit (Qiagen) column using a procedure recommended by the manufacturer (control) was compared with that purified using a modified procedure. The modification consisted in passing 100 µl mineral oil through the column after each change of the buffers (100 µl oil was passed through the column three times: after the DNA containing lysis buffer, after the "Wash 1" washing buffer, and after the "Wash 2" washing buffer). The final quantity of the eluted DNA was the same, irrespective of the method used - the average Ct (i.e., the threshold cycle in the quantitative PCR) value from 8 replicates was 19.68 for the control, and 19.24 for the modified procedure. It means that the membrane-bound DNA is not released from the membrane during the washing with oil.

### The use of mineral oil successfully replaces the necessity of drying the column prior to elution

The quantity of plasmid DNA purified with a QIAamp DNA mini kit (Qiagen) column using a procedure recommended by the manufacturer (control) was compared with that purified using a modified procedure. The modification consisted in replacing the column drying step prior to the elution by washing it with 300 µl mineral oil. The final quantity of the eluted DNA was the same, irrespective of the method used and the concentration of the target DNA (the average Ct value from 3 replicates was 19.62 for the control and 18.91 for the modified procedure).

The same experiment was also performed using silicone oil instead of mineral oil, and the results indicated that also that type of hydrophobic fluid did not affect the purification efficiency.

Even when a small quantity of DNA was purified, for example 100 plasmid copies only, it was detectable in the eluate in each instance. This result is of paramount importance, as it allows for elimination of the most time consuming step of the nucleic acid purification procedure. Manufacturers of isolation kits recommend centrifugation of a column for at least two minutes in order to remove the residual alcohol and/or chaotropic salts from the column. When present in the eluate, they inhibit biochemical reactions such as PCR or restriction enzyme digestion. The experiments carried out by the inventors showed that a PCR reaction comprising as little as less than 10 % vol. of the washing buffer is totally inhibited.

### The flow method of DNA purification is significantly faster and at least equally efficient as compared with the minicolumn method

The quantity of genomic DNA purified with a Genomic Mini column (A&A Biotechnology) was compared with DNA purified with the flow method according to the invention (in accordance with the above description). The final quantity of the eluted DNA was the same, irrespective of the method used and the concentration of the target DNA, the time needed to accomplish the purification with the two methods was, however, dramatically different. The DNA purification with the standard minicolumn method takes over 10 minutes, whereas the method according to the present invention takes one and a half minute only. The efficiency of so extremely fast DNA isolation using the buffers and bed provided by A&A Biotechnology amounts to approximately 50%, irrespective of the initial DNA concentration and is equally high as in the traditional minicolumn method. The flow method of DNA purification is equally efficient for different quantities of the initial DNA. Fig. 3 shows a diagram presenting the Ct values (threshold cycle - a value decreasing with increasing initial DNA quantity) for real-time PCR reactions performed on diluted genomic DNA from *E. coli* before and after purification with the flow method.

### 2.3 RNA isolation

The use of a mineral oil to wash out the residual washing buffers from the bed may also be applied in RNA isolation

The quantity of RNA purified with a Viral RNA Mini Kit (Syngen) column using a procedure recommended by the manufacturer (control) was compared with that purified using a modified procedure, consisting in replacing the column drying step prior to the elution by washing it with 300 µl mineral oil. The recovered quantity of RNA did not differ depending on the method used (Ct = 15.1 for the control; Ct = 15.2 for the isolation with oil).

The flow method of RNA purification with the method according to the present invention is significantly faster and at least equally efficient as compared with the minicolumn method.

The flow method employing the method according to the present invention is also very efficient in purification of small quantities of RNA. Even when only 100 RNA copies were subjected to purification, each time it was detected in the eluate.

### 2.4 Isolation of RNA and DNA mixture

The method using a hydrophobic fluid to wash out the residual washing buffers from the bed was also successfully tested for isolation of a mixture comprising DNA and RNA. The quantities of DNA and RNA co-purified with a Viral RNA Mini Kit (Syngen) column using a procedure recommended by the manufacturer were compared with those obtained with a modified procedure, consisting in replacing the column drying step prior to the elution by washing it with 300 µl mineral oil.

Both types of nucleic acids were recovered with the same efficiency, irrespective of the method used.

The method using a hydrophobic fluid is effective in the isolation of nucleic acids originating from different sources.

Numerous experiments have been carried out to purify nucleic acids from bacterial samples (E. *coli*, *M. smegmatis, B. subtilis*) and lentiviruses, usually with addition of fresh or frozen whole blood. Regardless of whether the isolation was performed in minicolumns or with the flow method shown in Fig. 2, the purified nucleic acids remained pure to the extent that they could be used in the real-time PCR reactions, and in the case of RNA, also in the reverse transcription.

### List of terms

- 1: conduit
- 2: conveying means
- 3: bed
- 4: opening
- 5: first end of the conduit
- 6: second end of the conduit
- 11: first part of the conduit
- 12: second part of the conduit
- 21: hydrophobic fluid
- 22: sample
- 23: washing fluid
- 24: elution fluid

Arrows and letter references a-g indicate flow direction of the fluids.

## Claims

1. A method of isolation and/or purification and/or concentration of nucleic acids, in particular from biological samples, performed in flow within a conduit, comprising steps of nucleic acid binding on a bed, bed washing and elution of the bound nucleic acids, **characterised in that** the nucleic acids are being bound to a bed (3) placed in the lumen of the conduit (1), stationary relative to the conduit (1) and occupying the entire cross section of the conduit, wherein the working fluids, including at least a washing fluid (23) and an elution fluid (24), are passed through the bed (3) in portions separated from each other by a hydrophobic fluid (21) immiscible with them, wherein the hydrophobic fluid (21), while passing through the bed, washes out the residual preceding fluid, in particular the washing fluid (23) prior to the elution step, wherein the method does not require the bed (3) drying step, and wherein the flow in the conduit (1) is provided by conveying means (2), and
wherein hydrophobic fluid is selected from mineral oil, silicone oil, hexadecane, paraffin oil, fluorinert and mixtures thereof,
wherein preferably the conduit is a capillary or a microfluidic conduit.

2. The method according to claim 1, **characterised in that** the nucleic acids are contained in structures surrounded by biological membranes, for example in cells or organelles, and the sample is preferably lysed prior to the binding of nucleic acids to the bed (3).

3. The method according to claim 1, **characterised in that** the following is passed through the bed (3) in the order as follows:
- sample (22) comprising released nucleic acids in the form able to bind to the bed,
- then, optionally, a portion of a hydrophobic fluid (21),
- then other working fluids, including at least:
• washing fluid or fluids (23),
• a portion of a hydrophobic fluid (21), required to remove residual washing fluid(s) from the bed,
• elution fluid (24),
• optionally the last portion of the hydrophobic fluid (21).

4. The method according to any one of the preceding claims, **characterised in that** the hydrophobic fluid (21) is a mineral oil and/or silicone oil.

5. The method according to any one of claims 1-4, **characterised in that** the conveying means (2) are provided by overpressure provided from the first end (5) of the conduit (1), especially in the form of a pump, preferably a syringe pump, and/or underpressure provided from the second end (6) of the conduit (1).

6. The method according to any one of claims 1-5, **characterised in that** the conveying means (2) are provided by centrifugation, wherein the first part (11) of the conduit (1) is located the closest to the axis of rotation.

7. The method according to any one of the preceding claims, **characterised in that** the material binding nucleic acids is used as the bed (3), preferably glass, borosilicate and/or silica material, material in amorphous form, as fibres, particles, beads, granules, or any other form, advantageous for binding of nucleic acids.

8. A system for isolation and/or purification and/or concentration of nucleic acids, in particular from biological samples, with the method defined in any one of claims 1-7, **characterised in that** it comprises:
- a conduit (1), comprising the first part (11) and the second part (12) of the conduit (1),
- conveying means (2), and
- a nucleic acid binding bed (3) placed in the lumen of the conduit (1) between the first part (11) and the second part (12) of the conduit (1), stationary relative to the conduit (1) and occupying the entire cross section of the conduit,
wherein the system utilizes working fluids for purification, isolation and/or concentration of nucleic acids, including at least a washing fluid orfluids (23), and an elution fluid (24), wherein said fluids are used in portions separated from each other by a hydrophobic fluid (21) immiscible with them,
wherein the hydrophobic fluid is selected from mineral oil, silicone oil, hexadecane, paraffin oil, fluorinert and mixtures thereof, and
wherein preferably the conduit is a capillary conduit.

9. The system according to claim 8, **characterised in that** the first part (11) of the conduit (1) represents a reservoir for working fluids, and said working fluids, including at least a washing fluid (23), a hydrophobic fluid (21) immiscible with the washing fluid, and an elution fluid (24) immiscible with the hydrophobic fluid, can be placed together in a working reservoir, preferably located in the first part (11) of the conduit (1), in the form of portions, separated from each other by the hydrophobic fluid (21) immiscible with them.

10. The system according to any one of claims 8-9, **characterised in that** the hydrophobic fluid (21) is a mineral oil and/or silicone oil.

11. The system according to any one of claims 8-10, **characterised in that** the conveying means (2) are provided by overpressure provided from the first end (5) of the conduit (1), especially in the form of a pump, preferably a syringe pump, and/or underpressure provided from the second end (6) of the conduit (1).

12. The system according to any one of claims 8-10, **characterised in that** the conveying means (2) are provided by centrifugation, wherein the first part (11) of the conduit (1) is located the closest to the axis of rotation.

13. The system according to any one of claims 8-12, **characterised in that** it enables one-way flow through the bed (3), through which the working fluids are passed in sequence in portions separated by a hydrophobic fluid (21) immiscible with them, including in particular a washing fluid or fluids (23) and an elution fluid (24) separated from each other by a hydrophobic fluid (21) immiscible with them, which washes out the residual washing fluid or fluids (23) from the bed (3).

14. The system according to any one of claims 8-12, **characterised in that** it enables two-way flow through the bed (3), so that a sample (22) comprising nucleic acids, lysed if required, is passed through the bed (3) in one direction (a), and then again in the opposite direction (b), and subsequently other working fluids are passed through the bed (3) in portions separated from each other by a hydrophobic fluid (21) immiscible with them, including in particular a washing fluid or fluids (23) and an elution fluid (24) separated from each other by a hydrophobic fluid (21) immiscible with them, which washes out residual washing fluid orfluids (23) from the bed (3).

15. The system according to any of claims 8-14, **characterised in that**
it allows for delivering a sample (22), comprising nucleic acids, in front of the bed (3) through an opening, preferably through a side conduit; or
it allows for delivering a sample (22) and individual working fluids and a hydrophobic fluid (21) immiscible with them, through separate conduits.

## Patentansprüche

1. Verfahren zur Isolierung und/oder Reinigung und/oder Konzentration von Nukleinsäuren, insbesondere aus biologischen Proben, das in der Strömung innerhalb einer Leitung durchgeführt wird, umfassend die Schritte der Nukleinsäurebindung auf einem Bett, des Bettwaschens und der Elution der gebundenen Nukleinsäuren, **dadurch gekennzeichnet, dass** die Nukleinsäuren an ein Bett (3) gebunden werden, das im Lumen der Leitung (1) angeordnet ist, relativ zur Leitung (1) stationär ist und den gesamten Querschnitt der Leitung einnimmt, wobei die Arbeitsfluide, die mindestens ein Waschfluid (23) und ein Elutionsfluid (24) umfassen, durch das Bett (3) in Portionen durchgelaufen werden, die durch ein mit ihnen nicht mischbares hydrophobes Fluid (21) voneinander getrennt sind, wobei das hydrophobe Fluid (21) während der Strömung durch das Bett das restliche vorhergehende Fluid, insbesondere das Waschfluid (23), vor dem Elutionsschritt auswäscht, wobei das Verfahren keinen Trocknungsschritt des Bettes (3) erfordert und wobei die Strömung in der Leitung (1) durch Fördermittel (2) bereitgestellt wird, und
wobei das hydrophobe Fluid aus Mineralöl, Silikonöl, Hexadecan, Paraffinöl, Fluorinert und Mischungen davon ausgewählt ist,
wobei die Leitung vorzugsweise eine kapillare oder eine mikrofluidische Leitung ist.

2. Verfahren nach Anspruch 1, **dadurch gekennzeichnet, dass** die Nukleinsäuren in Strukturen umgebend von biologischen Membranen enthalten sind, beispielsweise in Zellen oder Organellen, und die Probe vor der Bindung der Nukleinsäuren an das Bett (3) vorzugsweise lysiert wird.

3. Verfahren nach Anspruch 1, **dadurch gekennzeichnet, dass** durch das Bett (3) in der folgenden Reihenfolge durchgelaufen werden:
- Probe (22), die freigesetzte Nukleinsäuren in einer Form enthält, die in der Lage ist, an das Bett zu binden,
- dann gegebenenfalls eine Portion eines hydrophoben Fluids (21),
- dann andere Arbeitsfluide, umfassend mindestens:
• Waschfluid oder Waschfluide (23),
• eine Portion eines hydrophoben Fluids (21), das zur Entfernung von Waschfluid(e) aus dem Bett erforderlich ist,
• Elutionsfluid (24),
• gegebenenfalls eine letzte Portion eines hydrophoben Fluids (21).

4. Verfahren nach einem der vorstehenden Ansprüche, **dadurch gekennzeichnet, dass** das hydrophobe Fluid (21) ein Mineralöl und/oder Silikonöl ist.

5. Verfahren nach einem der Ansprüche 1-4, **dadurch gekennzeichnet, dass** die Fördermittel (2) durch Überdruck, der von dem ersten Ende (5) der Leitung (1), insbesondere in Form einer Pumpe, vorzugsweise einer Spritzenpumpe, bereitgestellt werden, und/oder durch Unterdruck, der von dem zweiten Ende (6) der Leitung (1) bereitgestellt werden.

6. Verfahren nach einem der Ansprüche 1-5, **dadurch gekennzeichnet, dass** die Fördermittel (2) durch Zentrifugieren bereitgestellt werden, wobei der erste Teil (11) der Leitung (1) am nächsten an der Achse angeordnet ist.

7. Verfahren nach einem der vorstehenden Ansprüche, **dadurch gekennzeichnet, dass** das Material, das Nukleinsäuren bindet, als Bett (3) verwendet wird, vorzugsweise Glas-, Borsilikat- und/oder Silika-Material, Material in amorpher Form, als Fasern, Partikel, Perlen, Granulat oder in jeder anderen Form, die für die Bindung von Nukleinsäuren vorteilhaft ist.

8. System zur Isolierung und/oder Reinigung und/oder Konzentration von Nukleinsäuren, insbesondere aus biologischen Proben, mit dem in einem der Ansprüche 1-7 definierten Verfahren, **dadurch gekennzeichnet, dass** es umfasst:
- eine Leitung (1), die den ersten Teil (11) und den zweiten Teil (12) der Leitung (1) umfasst,
- Fördermittel (2), und
- ein Nukleinsäure bindendes Bett (3), das im Lumen der Leitung (1) zwischen dem ersten Teil (11) und dem zweiten Teil (12) der Leitung (1) angeordnet ist, stationär in Bezug auf die Leitung (1) ist und den gesamten Querschnitt der Leitung einnimmt,
wobei das System Arbeitsfluide zur Reinigung, Isolierung und/oder Konzentration von Nukleinsäuren verwendet, einschließlich mindestens ein Waschfluid oder Waschfluide (23) und ein Elutionsfluid (24), wobei die genannte Fluide in Portionen verwendet werden, die durch ein hydrophobes Fluid (21), das mit ihnen nicht mischbar ist, voneinander getrennt sind,
wobei das hydrophobe Fluid aus Mineralöl, Silikonöl, Hexadecan, Paraffinöl, Fluorinert und Mischungen davon ausgewählt ist, und
wobei die Leitung vorzugsweise eine kapillare Leitung ist.

9. System nach Anspruch 8, **dadurch gekennzeichnet, dass** der erste Teil (11) der Leitung (1) ein Reservoir für Arbeitsfluide darstellt, und die genannten Arbeitsfluide, die mindestens ein Waschfluid (23), ein mit dem Waschfluid ein nicht mischbares hydrophobes Fluid (21) und ein mit dem hydrophoben Fluid nicht mischbares Elutionsfluid (24) umfassen, zusammen in einem Arbeitsreservoir, das vorzugsweise im ersten Teil (11) der Leitung (1) angeordnet ist, in Form von Portionen angeordnet werden können, die durch das mit ihnen nicht mischbare hydrophobe Fluid (21) voneinander getrennt sind.

10. System nach einem der Ansprüche 8-9, **dadurch gekennzeichnet, dass** das hydrophobe Fluid (21) ein Mineralöl und/oder Silikonöl ist.

11. System nach einem der Ansprüche 8-10, **dadurch gekennzeichnet, dass** die Fördermittel (2) durch Überdruck, der von dem ersten Ende (5) der Leitung (1), insbesondere in Form einer Pumpe, vorzugsweise einer Spritzenpumpe, bereitgestellt werden, und/oder durch Unterdruck, der von dem zweiten Ende (6) der Leitung (1) bereitgestellt werden.

12. System nach einem der Ansprüche 8-10, **dadurch gekennzeichnet, dass** die Fördermittel (2) durch Zentrifugieren bereitgestellt werden, wobei der erste Teil (11) der Leitung (1) am nächsten an der Achse angeordnet ist.

13. System nach einem der Ansprüche 8-12, **dadurch gekennzeichnet, dass** es eine Einwegströmung durch das Bett (3) ermöglicht, durch das die Arbeitsfluide nacheinander in Portionen durchgelaufen werden, die durch ein nicht mit ihnen mischbares hydrophobes Fluid (21) getrennt sind, insbesondere ein Waschfluid oder Waschfluide (23) und eine Elutionsfluid (24), die durch ein nicht mit ihnen mischbares hydrophobes Fluid (21) voneinander getrennt sind, das das(die) restliche(n) Waschfluid oder Waschfluide (23) aus dem Bett (3) auswaschen.

14. Das System nach einem der Ansprüche 8-12, **dadurch gekennzeichnet, dass** es eine Zweiwegströmung durch das Bett (3) ermöglicht, so dass eine Probe (22), die Nukleinsäuren umfasst, die gegebenenfalls lysiert sind, durch das Bett (3) in einer Richtung (a) und dann wieder in der entgegengesetzten Richtung (b) in Portionen, die durch ein mit ihnen nicht mischbares hydrophobes Fluid (21) voneinander getrennt sind, durch das Bett (3) durchgelaufen werden, einschließend insbesondere ein Waschfluid oder Waschfluide (23) und ein Elutionsfluid (24), die durch ein mit ihnen nicht mischbares hydrophobes Fluid (21) voneinander getrennt sind und die restliches Waschfluid oder Waschfluide (23) aus dem Bett (3) auswaschen.

15. System nach einem der Ansprüche 8-14, **dadurch gekennzeichnet, dass**
es ermöglicht die Ablieferung einer Nukleinsäuren umfassenden Probe (22) vor dem Bett (3) durch eine Öffnung, vorzugsweise durch eine Seitenleitung; oder
es ermöglicht die Ablieferung einer Probe (22) und einzelner Arbeitsfluide und eines damit nicht mischbaren hydrophoben Fluid (21) durch separate Leitungen.

## Revendications

1. Une méthode d'isolement et/ou de purification, et/ou de concentration d'acides nucléiques, en particulier à partir d'échantillons biologiques, effectuée en écoulement dans un conduit, comprenant des étapes de liaison d'acides nucléiques sur un lit, de lavage de lit et d'élution des acides nucléiques liés, **caractérisée en ce que** les acides nucléiques sont liés à un lit (3) placé dans la lumière du conduit (1), stationnaire par rapport au conduit (1) et occupant toute la section transversale du conduit, dans laquelle les fluides de travail, comprenant au moins un fluide de lavage (23) et un fluide d'élution (24), sont passés à travers le lit (3) en portions séparées les unes des autres par un fluide hydrophobe (21) immiscible avec eux, dans laquelle le fluide hydrophobe (21), en traversant le lit, lave le fluide précédent résiduel, en particulier le fluide de lavage (23) avant l'étape d'élution, dans laquelle la méthode ne nécessite pas l'étape de séchage du lit (3), et dans laquelle l'écoulement dans le conduit (1) est assuré par des moyens de transport (2),
dans laquelle le fluide hydrophobe est choisi parmi l'huile minérale, l'huile de silicone, l'hexadécane, l'huile de paraffine, le Fluorinert et leurs mélanges,
dans laquelle de préférence le conduit est un conduit capillaire ou microfluidique.

2. La méthode selon la revendication 1, **caractérisée en ce que** les acides nucléiques sont contenus dans des structures entourées de membranes biologiques, par exemple dans des cellules ou des organelles, et l'échantillon est de préférence lysé avant la liaison des acides nucléiques au lit (3).

3. La méthode selon la revendication 1, **caractérisée en ce que** ce qui suit est passé à travers le lit (3) dans l'ordre suivant :
- échantillon (22) comprenant des acides nucléiques libérés sous forme capable de se lier au lit,
- puis, éventuellement, une portion d'un fluide hydrophobe (21),
- puis d'autres fluides de travail, y compris au moins :
• fluide ou fluides de lavage (23),
• une portion d'un fluide hydrophobe (21), nécessaire pour éliminer le ou les fluides de lavage résiduels du lit,
• fluide d'élution (24),
- éventuellement la dernière portion du fluide hydrophobe (21).

4. La méthode selon l'une quelconque des revendications précédentes, **caractérisée en ce que** le fluide hydrophobe (21) est une huile minérale et/ou une huile de silicone.

5. La méthode selon l'une quelconque des revendications 1-4, **caractérisée en ce que** les moyens de transport (2) sont assurés par une surpression fournie par la première extrémité (5) du conduit (1), notamment sous la forme d'une pompe, de préférence une pompe à seringue, et/ou une sous-pression fournie par la seconde extrémité (6) du conduit (1).

6. La méthode selon l'une quelconque des revendications 1-5, **caractérisée en ce que** les moyens de transport (2) sont fournis par centrifugation, la première partie (11) du conduit (1) étant située le plus près de l'axe de rotation.

7. La méthode selon l'une quelconque des revendications précédentes, **caractérisée en ce que** le matériau liant les acides nucléiques est utilisé comme lit (3), de préférence verre, borosilicate et/ou silice, matériau sous forme amorphe, sous forme de fibres, particules, billes, granulés ou toute autre forme, avantageux pour la liaison des acides nucléiques.

8. Un système d'isolement et/ou de purification, et/ou de concentration d'acides nucléiques, en particulier à partir d'échantillons biologiques, avec la méthode définie dans l'une quelconque des revendications 1-7, **caractérisé en ce qu'il** comprend :
- un conduit (1), comprenant la première partie (11) et la seconde partie (12) du conduit (1),
- des moyens de transport (2), et
- un lit de liaison d'acides nucléiques (3) placé dans la lumière du conduit (1) entre la première partie (11) et la seconde partie (12) du conduit (1), stationnaire par rapport au conduit (1) et occupant toute la section transversale du conduit,
dans lequel le système utilise des fluides de travail pour la purification, l'isolement et/ou la concentration d'acides nucléiques, y compris au moins un ou des fluides de lavage (23) et un fluide d'élution (24), dans lequel lesdits fluides sont utilisés en portions séparées les unes des autres par un fluide hydrophobe (21) immiscible avec eux,
dans lequel le fluide hydrophobe est choisi parmi l'huile minérale, l'huile de silicone, l'hexadécane, l'huile de paraffine, le Fluorinert et leurs mélanges, et
dans lequel de préférence le conduit est un conduit capillaire.

9. Le système selon la revendication 8, **caractérisé en ce que** la première partie (11) du conduit (1) représente un réservoir pour les fluides de travail, et lesdits fluides de travail, y compris au moins un fluide de lavage (23), un fluide hydrophobe (21) immiscible avec le fluide de lavage, et un fluide d'élution (24) immiscible avec le fluide hydrophobe, peuvent être placés ensemble dans un réservoir de travail, de préférence situé dans la première partie (11) du conduit (1), sous forme de portions, séparées les unes des autres par le fluide hydrophobe (21) immiscible avec eux.

10. Le système selon l'une quelconque des revendications 8-9, **caractérisé en ce que** le fluide hydrophobe (21) est une huile minérale et/ou une huile de silicone.

11. Le système selon l'une quelconque des revendications 8-10, **caractérisé en ce que** les moyens de transport (2) sont assurés par une surpression fournie par la première extrémité (5) du conduit (1), notamment sous la forme d'une pompe, de préférence une pompe à seringue, et/ou une sous-pression fournie par la seconde extrémité (6) du conduit (1).

12. Le système selon l'une quelconque des revendications 8-10, **caractérisé en ce que** les moyens de transport (2) sont fournis par centrifugation, la première partie (11) du conduit (1) étant située le plus près de l'axe de rotation.

13. Le système selon l'une quelconque des revendications 8-12, **caractérisé en ce qu'**il permet un écoulement unidirectionnel à travers le lit (3), à travers lequel les fluides de travail sont passés en séquence en portions séparées par un fluide hydrophobe (21) immiscible avec eux, y compris notamment un ou des fluides de lavage (23) et un fluide d'élution (24) séparé les uns des autres par un fluide hydrophobe (21) immiscible avec eux, qui lave le ou les fluides de lavage résiduels (23) à partir du lit (3).

14. Le système selon l'une quelconque des revendications 8-12, **caractérisé en ce qu'**il permet un écoulement bidirectionnel à travers le lit (3), de sorte qu'un échantillon (22) comprenant des acides nucléiques, lysés si nécessaire, traverse le lit (3) dans une direction (a), et puis à nouveau dans la direction opposée (b), et par la suite d'autres fluides de travail sont passés à travers le lit (3) en portions séparées les unes des autres par un fluide hydrophobe (21) immiscible avec eux, comprenant notamment un ou des fluides de lavage (23) et un fluide d'élution (24) séparés les uns des autres par un fluide hydrophobe (21) immiscible avec eux, qui lave le ou les fluides de lavage résiduels (23) à partir du lit (3).

15. Le système selon l'une quelconque des revendications 8-12, **caractérisé en ce que**
il permet de délivrer un échantillon (22), comprenant des acides nucléiques, devant le lit (3) à travers une ouverture, de préférence à travers un conduit latéral ; ou
il permet de délivrer un échantillon (22) et des fluides de travail individuels et un fluide hydrophobe (21) immiscible avec eux, à travers des conduits séparés.
